# EUROPEAN PATENT SPECIFICATION

(11) **EP 2 721 154 B1**
(45) Date of publication and mention of the grant of the patent: **02.01.2019**
(21) Application number: 12735365.4
(22) Date of filing: 15.06.2012
(51) Int. Cl.: C12N 15/10

(54) **METHODS FOR PREPARATIVE IN VITRO CLONING**
VERFAHREN FÜR PRÄPARATIVES IN-VITRO-KLONEN
PROCÉDÉS DE PRÉPARATION DE CLONAGE IN VITRO

(30) Priority: 15.06.2011 US 201161497506 P
(43) Date of publication of application: 23.04.2014
(60) Divisional application: 18207413.8
(73) Proprietor: Gen9, Inc., Boston MA 02210 (US)
(72) Inventor: KUNG, Li-Yun A., Arlington, MA 02474 (US); SCHINDLER, Daniel, Newton, MA 02464 (US); JACOBSON, Joseph, Newton, MA 02459 (US)
(74) Representative: Sutcliffe, Nicholas Robert
(86) International application number: PCT/US2012/042597
(87) International publication number: WO 2012/174337

(56) References cited:
- US-A- 5 604 097
- PETRIK J ET AL: "Advances in transfusion medicine in the first decade of the 21st century: Advances in miniaturized technologies", TRANSFUSION AND APHERESIS SCIENCE, vol. 45, no. 1, 11 June 2011 (2011-06-11), pages 45-51, XP028269308, ISSN: 1473-0502, DOI: 10.1016/J.TRANSCI.2011.06.002 [retrieved on 2011-06-11]
- MCCLAIN MARK S ET AL: "Genome sequence analysis of Helicobacter pylori strains associated with gastric ulceration and gastric cancer", BMC GENOMICS, BIOMED CENTRAL LTD, LONDON, UK, vol. 10, no. 1, 5 January 2009 (2009-01-05), page 3, XP021047945, ISSN: 1471-2164, DOI: 10.1186/1471-2164-10-3
- VOGELSTEIN BERT ET AL: "Digital PCR", PROCEEDINGS OF THE NATIONAL ACADEMY OF SCIENCES, NATIONAL ACADEMY OF SCIENCES, US, vol. 96, no. 16, 3 August 1999 (1999-08-03), pages 9236-9241, XP002185144, ISSN: 0027-8424, DOI: 10.1073/PNAS.96.16.9236
- RAMACHANDRAN S ET AL: "End-point limiting-dilution real-time PCR assay for evaluation of hepatitis C virus quasispecies in serum: Performance under optimal and suboptimal conditions", JOURNAL OF VIROLOGICAL METHODS, ELSEVIER BV, NL, vol. 151, no. 2, 1 August 2008 (2008-08-01), pages 217-224, XP022832552, ISSN: 0166-0934, DOI: 10.1016/J.JVIROMET.2008.05.005 [retrieved on 2008-06-20]
- Frank Mccaughan ET AL: "Single-molecule genomics", The Journal of Pathology, 1 January 2009 (2009-01-01), pages n/a-n/a, XP55027790, ISSN: 0022-3417, DOI: 10.1002/path.2647

## Description

### FIELD OF THE INVENTION

Methods are provided herein relate to the selection of a target nucleic acid sequence from a population of nucleic acid sequences. More particularly methods are provided for isolation of target sequences nucleic acid sequences of interest away from a library of nucleic acid sequences.

### BACKGROUND

Methods for cloning nucleic acids and separating target sequences away from closely related but non-target sequences have been staple molecular biology tools. These common methods typically involve incorporation of the mixture of sequences with prepared vector pieces to prepare plasmids. The plasmids are then introduced into bacterial cells (transformation), which typically confer an antibiotic resistance to the strain. Selection of cells of the strain and proper dilution of the cells onto agar plates allows for identification of colonies arising from individual bacterial cells carrying individual sequences from the initial mixture. Subsequent manipulation of these colonies allow for the identification of target sequences away from the non-target sequences in the pool.

These common methods have drawbacks. The process is slow, with transformation of a bacterial strain and subsequent colony growth typically taking place overnight. Each colony must then be individually isolated and either grown in liquid media. Another drawback is that the transformation is relatively expensive.

The limiting dilution technique has been used to isolate single cells from a pooled suspension of cells, as well as for counting of DNA molecules. The basic method involves making a measurement of the number of cells or the concentration of DNA, diluting to very low concentration, and aliquoting into separate wells such that the number of cells or DNA molecules is less than one per well. Subsequent cell division or DNA amplification (e.g. by PCR) should reveal a Poissonian distribution of the wells corresponding to wells which were initially empty, seeded with one cell or molecule, and those seeded with multiples.

### SUMMARY

Aspects of the invention relate to the isolation of target nucleic acid molecules having a predefined sequence as set out in claim 1. In some disclosure herein, the method comprises the steps of providing a population of nucleic acid molecules comprising a plurality of distinct nucleic acid molecules; providing a plurality of oligonucleotide tags; attaching the oligonucleotide tags to one end terminus of the plurality nucleic acid molecules thereby generating a subpopulation of nucleic acid-oligonucleotide tag molecules; amplifying the plurality of nucleic acid-oligonucleotide tag molecules using primers complementary to the plurality of oligonucleotide tags; and isolating at least one target nucleic acid-oligonucleotide tag molecule of the subpopulation of nucleic acid-oligonucleotide tag molecules.

In some disclosure herein, the method further comprises sequencing the at least one isolated target nucleic acid-oligonucleotide tag molecule and identifying the target nucleic acid. In some disclosure herein, the sequencing step is by high throughput sequencing. In some disclosure herein, the method further comprises amplifying the at least one target nucleic acid-oligonucleotide tag molecule.

In some disclosure herein, the step of isolating is by limiting dilution. In some disclosure herein, the target nucleic acid-oligonucleotide tag molecule is amplified.

Some disclosure relates to a method of isolating a target nucleic acid, comprising (a) providing a population comprising a plurality of different nucleic acid molecules; (b) providing a plurality of microparticles, wherein each microparticle has an oligonucleotide sequence complementary to a portion of the nucleic acid molecules immobilized on its surface; (c) forming a population of nucleic acid molecules hybridized to the complementary oligonucleotide sequence on the microparticles; and (d) isolating at least one target nucleic acid molecule. In some disclosure herein, each microparticle has a single complementary sequence on its surface. In some disclosure herein, the complementary oligonucleotide sequences are identical. Yet in other disclosure herein, the complementary oligonucleotide sequences are different. In some disclosure herein, the plurality of nucleic acid molecules comprises an oligonucleotide tag at one terminus. In some disclosure herein, the plurality of nucleic acid molecules can have the same oligonucleotide tag or a different oligonucleotide tag. In some disclosure herein, the step of isolating is by limiting dilution. In some disclosure herein, the method further comprises amplifying the at least one target molecule.

Some disclosure relates to a method of isolating a target nucleic acid, the method comprising (a) providing a population comprising a plurality of different nucleic acid molecules; (b) providing a dilution of the population of nucleic acid molecules; (c) separating the nucleic acid molecules into samples comprising a single molecule or a smaller number of molecules; (d) amplifying the single molecules thereby forming amplified nucleic acid molecules; (e) optionally repeating step (c) and (d) in samples that do not comprise a nucleic acid molecule; and (f) isolating at least one target nucleic acid molecule. In some disclosure herein, the target nucleic acid molecule has a predefined sequence. In some disclosure herein, the method further comprises sequencing the at least one target nucleic acid molecule.

### BRIEF DESCRIPTION OF THE DRAWINGS

Fig. 1 illustrates a non-limiting exemplary method of in vitro cloning by increasing the limiting dilution.
Fig. 2 illustrates a non-limiting exemplary method of in vitro cloning using beads with single ligand sites for nucleic acid attachment.
Fig. 3 illustrates a non-limiting exemplary method of in vitro cloning using a library of barcode sequences.

### DETAILED DESCRIPTION OF THE INVENTION

Disclosure herein relates to methods and compositions for analyzing and separating at least one nucleic acid having a predefined sequence from a pool comprising a plurality of different nucleic acid sequences. In some disclosure herein, the pool of nucleic acid sequences comprises variants of the nucleic acid sequence of interest and/or nucleic acid sequences having a similar length than the nucleic acid sequence of interest. Disclosure herein is particularly useful for isolating nucleic acids sequence of interest from a population of nucleic acid sequences such as a library of nucleic acid sequences.

Aspects of the technology provided herein are useful for increasing the accuracy, yield, throughput, and/or cost efficiency of nucleic acid synthesis and assembly reactions. In some disclosure herein, the methods disclosed herein are particularly useful for isolating nucleic acid sequences for assembly of nucleic acid molecules having a predefined sequence. As used herein the terms "nucleic acid", "polynucleotide", "oligonucleotide" are used interchangeably and refer to naturally-occurring or synthetic polymeric forms of nucleotides. The oligonucleotides and nucleic acid molecules of the present disclosure may be formed from naturally occurring nucleotides, for example forming deoxyribonucleic acid (DNA) or ribonucleic acid (RNA) molecules. Alternatively, the naturally occurring oligonucleotides may include structural modifications to alter their properties, such as in peptide nucleic acids (PNA) or in locked nucleic acids (LNA). The solid phase synthesis of oligonucleotides and nucleic acid molecules with naturally occurring or artificial bases is well known in the art. The terms should be understood to include equivalents, analogs of either RNA or DNA made from nucleotide analogs and as applicable to the embodiment being described, single-stranded or double-stranded polynucleotides. Nucleotides useful in the disclosure include, for example, naturally-occurring nucleotides (for example, ribonucleotides or deoxyribonucleotides), or natural or synthetic modifications of nucleotides, or artificial bases. As used herein, the term monomer refers to a member of a set of small molecules which are and can be joined together to from an oligomer, a polymer or a compound composed of two or more members. The particular ordering of monomers within a polymer is referred to herein as the "sequence" of the polymer. The set of monomers includes but is not limited to example, the set of common L-amino acids, the set of D-amino acids, the set of synthetic and/or natural amino acids, the set of nucleotides and the set of pentoses and hexoses. Disclosure described herein primarily with regard to the preparation of oligonucleotides, but could readily be applied in the preparation of other polymers such as peptides or polypeptides, polysaccharides, phospholipids, heteropolymers, polyesters, polycarbonates, polyureas, polyamides, polyethyleneimines, polyarylene sulfides, polysiloxanes, polyimides, polyacetates, or any other polymers.

As used herein, the term "predetermined sequence" or "predefined sequence" are used interchangeably and means that the sequence of the polymer is known and chosen before synthesis or assembly of the polymer. In particular, the disclosure is described herein primarily with regard to the preparation of nucleic acids molecules, the sequence of the oligonucleotide or polynucleotide being known and chosen before the synthesis or assembly of the nucleic acid molecules. In some disclosure of the technology provided herein, immobilized oligonucleotides or polynucleotides are used as a source of material. In various disclosure herein, the methods described herein use oligonucleotides, their sequence being determined based on the sequence of the final polynucleotides constructs to be synthesized. In one embodiment, oligonucleotides are short nucleic acid molecules. For example, oligonucleotides may be from 10 to about 300 nucleotides, from 20 to about 400 nucleotides, from 30 to about 500 nucleotides, from 40 to about 600 nucleotides, or more than about 600 nucleotides long. However, shorter or longer oligonucleotides may be used. Oligonucleotides may be designed to have different lengths. In some disclosure herein, the sequence of the polynucleotide construct may be divided up into a plurality of shorter sequences that can be synthesized in parallel and assembled into a single or a plurality of desired polynucleotide constructs using the methods described herein. In some disclosure herein, the assembly procedure may include several parallel and/or sequential reaction steps in which a plurality of different nucleic acids or oligonucleotides are synthesized or immobilized, primer-extended, and are combined in order to be assembled (e.g., by extension or ligation as described herein) to generate a longer nucleic acid product to be used for further assembly, cloning, or other applications.

In some disclosure herein, the nucleic acids molecules prepared according to the methods disclosed herein can be used for nucleic acid assembly and for assembly of libraries containing nucleic acids having predetermined sequence variations. Assembly strategies provided herein can be used to generate very large libraries representative of many different nucleic acid sequences of interest. In some disclosure herein, libraries of nucleic acids are libraries of sequence variants. Sequence variants may be variants of a single naturally-occurring protein encoding sequence. However, in some disclosure herein, sequence variants may be variants of a plurality of different protein-encoding sequences. Accordingly, one aspect of the technology provided herein relates to the assembling of precise high-density nucleic acid libraries. Aspects of the technology provided herein also provide precise high-density nucleic acid libraries. A high-density nucleic acid library may include more that 100 different sequence variants (e.g., about 10² to 10³; about 10³ to 10⁴; about 10⁴ to 10⁵; about 10⁵ to 10⁶; about 10⁶ to 10⁷; about 10⁷ to 10⁸; about 10⁸ to 10⁹; about 10⁹ to 10¹⁰; about 10¹⁰ to 10¹¹; about 10¹¹ to 10¹²; about 10¹² to 10¹³; about 10¹³ to 10¹⁴; about 10¹⁴ to 10¹⁵; or more different sequences) wherein a high percentage of the different sequences are specified sequences as opposed to random sequences (e.g., more than about 50%, more than about 60%, more than about 70%, more than about 75%, more than about 80%, more than about 85%, more than about 90%, about 91%, about 92%, about 93%, about 94%, about 95%, about 96%, about 97%, about 98%, about 99%, or more of the sequences are predetermined sequences of interest).

In some disclosure herein, the methods and devices provided herein use oligonucleotides that are immobilized on a surface or substrate (e.g., support-bound oligonucleotides). Support-bound oligonucleotides comprise for example, oligonucleotides complementary to construction oligonucleotides, anchor oligonucleotides and/or spacer oligonucleotides. As used herein the term "support", "substrate" and "surface" are used interchangeably and refers to a porous or non-porous solvent insoluble material on which polymers such as nucleic acids are synthesized or immobilized. As used herein "porous" means that the material contains pores having substantially uniform diameters (for example in the nm range). Porous materials include paper, synthetic filters etc. In such porous materials, the reaction may take place within the pores. The support can have any one of a number of shapes, such as pin, strip, plate, disk, rod, bends, cylindrical structure, particle, including bead, nanoparticles and the like. The support can have variable widths. The support can be hydrophilic or capable of being rendered hydrophilic. The support can include inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, ceramics, metals, and the like etc.; either used by themselves or in conjunction with other materials. In some disclosure herein, oligonucleotides are synthesized on an array format. For example, single-stranded oligonucleotides are synthesized in situ on a common support wherein each oligonucleotide is synthesized on a separate or discrete feature (or spot) on the substrate. In preferred disclosure herein, single-stranded oligonucleotides are bound to the surface of the support or feature. As used herein the term "array" refers to an arrangement of discrete features for storing, amplifying and releasing oligonucleotides or complementary oligonucleotides for further reactions. In a preferred embodiment, the support or array is addressable: the support includes two or more discrete addressable features at a particular predetermined location (i.e., an "address") on the support. Therefore, each oligonucleotide molecule of the array is localized to a known and defined location on the support. The sequence of each oligonucleotide can be determined from its position on the support. The array may comprise interfeatures regions. Interfeatures will typically not carry any oligonucleotide on their surface and will correspond to inert space.

In some disclosure herein, oligonucleotides are attached, spotted, immobilized, surface-bound, supported or synthesized on the discrete features of the surface or array. Oligonucleotides may be covalently attached to the surface or deposited on the surface. Arrays may be constructed, custom ordered or purchased from a commercial vendor (e.g., Agilent, Affymetrix, Nimblegen). Various methods of construction are well known in the art e.g., maskless array synthesizers, light directed methods utilizing masks, flow channel methods, spotting methods, etc. In some disclosure herein, construction and/or selection oligonucleotides may be synthesized on a solid support using maskless array synthesizer (MAS). Maskless array synthesizers are described, for example, in PCT application No. WO 99/42813 and in corresponding U.S. Pat. No. 6,375,903. Other examples are known of maskless instruments which can fabricate a custom DNA microarray in which each of the features in the array has a single-stranded DNA molecule of desired sequence. Other methods for synthesizing oligonucleotides include, for example, light-directed methods utilizing masks, flow channel methods, spotting methods, pin-based methods, and methods utilizing multiple supports. Light directed methods utilizing masks (e.g., VLSIPS™ methods) for the synthesis of oligonucleotides is described, for example, in U.S. Pat. Nos. 5,143,854, 5,510,270 and 5,527,681. These methods involve activating predefined regions of a solid support and then contacting the support with a preselected monomer solution. Selected regions can be activated by irradiation with a light source through a mask much in the manner of photolithography techniques used in integrated circuit fabrication. Other regions of the support remain inactive because illumination is blocked by the mask and they remain chemically protected. Thus, a light pattern defines which regions of the support react with a given monomer. By repeatedly activating different sets of predefined regions and contacting different monomer solutions with the support, a diverse array of polymers is produced on the support. Other steps, such as washing unreacted monomer solution from the support, can be optionally used. Other applicable methods include mechanical techniques such as those described in U.S. Pat. No. 5,384,261. Additional methods applicable to synthesis of oligonucleotides on a single support are described, for example, in U.S. Pat. No. 5,384,261. For example, reagents may be delivered to the support by either (1) flowing within a channel defined on predefined regions or (2) "spotting" on predefined regions. Other approaches, as well as combinations of spotting and flowing, may be employed as well. In each instance, certain activated regions of the support are mechanically separated from other regions when the monomer solutions are delivered to the various reaction sites. Flow channel methods involve, for example, microfluidic systems to control synthesis of oligonucleotides on a solid support. For example, diverse polymer sequences may be synthesized at selected regions of a solid support by forming flow channels on a surface of the support through which appropriate reagents flow or in which appropriate reagents are placed. Spotting methods for preparation of oligonucleotides on a solid support involve delivering reactants in relatively small quantities by directly depositing them in selected regions. In some steps, the entire support surface can be sprayed or otherwise coated with a solution, if it is more efficient to do so. Precisely measured aliquots of monomer solutions may be deposited dropwise by a dispenser that moves from region to region. Pin-based methods for synthesis of oligonucleotides on a solid support are described, for example, in U.S. Pat. No. 5,288,514. Pin-based methods utilize a support having a plurality of pins or other extensions. The pins are each inserted simultaneously into individual reagent containers in a tray. An array of 96 pins is commonly utilized with a 96-container tray, such as a 96-well microtiter dish. Each tray is filled with a particular reagent for coupling in a particular chemical reaction on an individual pin. Accordingly, the trays will often contain different reagents. Since the chemical reactions have been optimized such that each of the reactions can be performed under a relatively similar set of reaction conditions, it becomes possible to conduct multiple chemical coupling steps simultaneously.

In another embodiment, a plurality of oligonucleotides may be synthesized or immobilized on multiple supports. One example is a bead based synthesis method which is described, for example, in U.S. Pat. Nos. 5,770,358; 5,639,603; and 5,541,061. For the synthesis of molecules such as oligonucleotides on beads, a large plurality of beads is suspended in a suitable carrier (such as water) in a container. The beads are provided with optional spacer molecules having an active site to which is complexed, optionally, a protecting group. At each step of the synthesis, the beads are divided for coupling into a plurality of containers. After the nascent oligonucleotide chains are deprotected, a different monomer solution is added to each container, so that on all beads in a given container, the same nucleotide addition reaction occurs. The beads are then washed of excess reagents, pooled in a single container, mixed and redistributed into another plurality of containers in preparation for the next round of synthesis. It should be noted that by virtue of the large number of beads utilized at the outset, there will similarly be a large number of beads randomly dispersed in the container, each having a unique oligonucleotide sequence synthesized on a surface thereof after numerous rounds of randomized addition of bases. An individual bead may be tagged with a sequence which is unique to the double-stranded oligonucleotide thereon, to allow for identification during use.

Pre-synthesized oligonucleotide and/or polynucleotide sequences may be attached to a support or synthesized in situ using light-directed methods, flow channel and spotting methods, inkjet methods, pin-based methods and bead-based methods set forth in the following references: McGall et al. (1996) Proc. Natl. Acad. Sci. U.S.A. 93:13555; Synthetic DNA Arrays In Genetic Engineering, Vol. 20:111, Plenum Press (1998); Duggan et al. (1999) Nat. Genet. S21:10 ; Microarrays: Making Them and Using Them In Microarray Bioinformatics, Cambridge University Press, 2003; U.S. Patent Application Publication Nos. 2003/0068633 and 2002/0081582; U.S. Pat. Nos. 6,833,450, 6,830,890, 6,824,866, 6,800,439, 6,375,903 and 5,700,637; and PCT Publication Nos. WO 04/031399, WO 04/031351, WO 04/029586, WO 03/100012, WO 03/066212, WO 03/065038, WO 03/064699, WO 03/064027, WO 03/064026, WO 03/046223, WO 03/040410 and WO 02/24597. In some disclosure herein, pre-synthesized oligonucleotides are attached to a support or are synthesized using a spotting methodology wherein monomers solutions are deposited dropwise by a dispenser that moves from region to region (e.g., ink jet). In some disclosure herein, oligonucleotides are spotted on a support using, for example, a mechanical wave actuated dispenser.

In one aspect, the disclosure relates to a method for producing target polynucleotides having a predefined sequence on a solid support. In some disclosure herein, the synthetic polynucleotides can be at least about 1, 2, 3, 4, 5, 8, 10, 15, 20, 25, 30, 40, 50, 75, or 100 kilobases (kb), or 1 megabase (mb), or longer.

In some aspects, the disclosure relate to a method for the production of high fidelity polynucleotides. In exemplary disclosure herein, a composition of synthetic polynucleotides contains at least about 1%, 2%, 3%, 4%, 5%, 6%, 7%, 8%, 9%, 10%, 15%, 20%, 25%, 50%, 60%, 70%, 80%, 90%, 95 % or more, copies that are error free (e.g., having a sequence that does not deviate from a predetermined sequence). The percent of error free copies is based on the number of error free copies in the composition as compared to the total number of copies of the polynucleotide in the composition that were intended to have the correct, e.g., predefined or predetermined, sequence.

Some aspects the disclosure relate to the preparation of oligonucleotides for high fidelity polynucleotide assembly. Aspects of the disclosure may be useful to increase the throughput rate of a nucleic acid assembly procedure and/or reduce the number of steps or amounts of reagent used to generate a correctly assembled nucleic acid. In certain disclosure herein, aspects of the disclosure may be useful in the context of automated nucleic acid assembly to reduce the time, number of steps, amount of reagents, and other factors required for the assembly of each correct nucleic acid. Accordingly, these and other aspects of the disclosure may be useful to reduce the cost and time of one or more nucleic acid assembly procedures.

It should be appreciated that the sequence of a nucleic acid (or library of nucleic acids or assembled nucleic acids) may include some errors that may result from sequence errors introduced during the oligonucleotides synthesis, the synthesis of nucleic acids, and/or from assembly errors during the assembly reaction. In some disclosure herein, unwanted sequences may be present in some nucleic acids. For example, between 0% and 50% (e.g., less than 45%, less than 40%, less than 35%, less than 30%, less than 25%, less than 20%, less than 15%, less than 10%, less than 5% or less than 1%) of the sequences in a library may be unwanted sequences. In some disclosure herein, nucleic acid having the predefined (or correct sequence) can be selectively isolated. The term "selective isolation", as used herein, can involve physical isolation of a desired polynucleotide from others as by selective physical movement of the desired polynucleotide as well as selective inactivation, destruction, release, or removal of other polynucleotides than the polynucleotide of interest.

Devices and methods to selectively isolate the correct nucleic acid sequence from the incorrect nucleic acid sequences form a pool of nucleic acid sequences are provided herein. The correct sequence may be isolated by selectively isolating the correct sequence from the other incorrect sequences as by selectively isolating, moving or transferring the desired correct nucleic acid sequence. Alternatively, polynucleotides having an incorrect sequence can be selectively removed.

According to some methods of the disclosure, the nucleic acid sequences (construction oligonucleotides, assembly intermediates or desired assembled target nucleic acid) may first be diluted in order to obtain a clonal population of target polynucleotides (i.e. a population containing a single target polynucleotide sequence). As used herein, a "clonal nucleic acid" or "clonal population" or "clonal polynucleotide" are used interchangeably and refer to a clonal molecular population of nucleic acids, i.e. to nucleic acids or polynucleotide that are substantially or completely identical to each other. Accordingly, the dilution based protocol provides a population of nucleic acid molecules being substantially identical or identical to each other. In some disclosure herein, the polynucleotides are diluted serially. In some disclosure herein, the device integrates a serial dilution function. In some disclosure herein, the assembly product is serially diluted to produce a clonal population of nucleic acids. In some disclosure herein, the concentration and the number of molecules can be assessed prior to the dilution step and a dilution ratio is calculated in order to produce a clonal population. In an exemplary embodiment, the assembly product is diluted by a factor of at least 2, at least 3, at least 4, at least 5, at least 6, at least 7, at least 10, at least 20, at least 50, at least 100, at least 1,000 etc...

One of skill in the art will appreciate that the traditional limiting dilution techniques which follows the statistical Poisson distribution is often time consuming. As determined by the Poisson distribution, limiting dilution should result in a single-hit e.g. one clone per well. However, if the sample is very large, a variable number of clones (e.g. two-hit) can be present in a single well and multiple rounds are required in order to assure monoclonality. The disclosure relates to methods for preparative isolation of nucleic acid molecules in a more efficient way than the method of limiting dilution.

In some disclosure, the nucleic acid sequences are sorted by an improved limiting dilution technique. Specifically, in some disclosure herein, the methods use a feedback loop to increase the efficiency of separation beyond the Poisson distribution of limiting dilution. In some disclosure herein, the process disclosed herein is an iterative process. The process can use a fluorescence signal as a feedback mechanism for identification of empty samples, thereby allowing for more effective in vitro cloning than the Poisson limiting dilution.

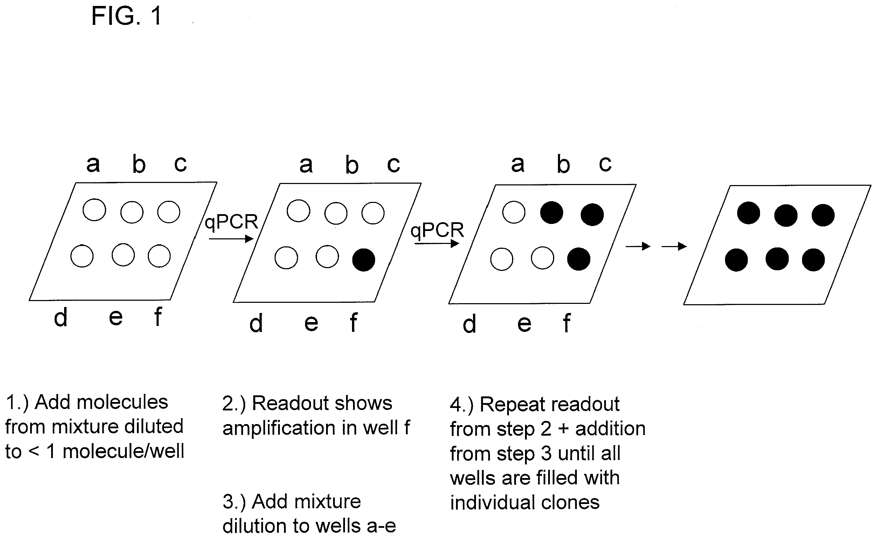

In some disclosure herein, a pool of nucleic acid sequences is diluted to form a diluted solution or mixture of nucleic acid molecules. The nucleic acid molecules are separated into samples comprising a single or a smaller number of molecules. For example, the diluted solution can be used for seeding the wells of a plate such that individual wells of a plate (e.g. 96 well plate) are filled with less than one molecule per well. Cycles of quantitative PCR (qPCR) reactions can be run on all the samples, allowing for identification of samples (e.g. wells) initially seeded with individual molecules. Presence of nucleic acid sequences within each sample can be determined by fluorescence. Because of the way the initial dilution is set, very few samples (e.g. well) would be initially seeded with multiple molecules. As illustrated in Figure 1, step 1, readout of the fluorescence shows the presence of amplification products in a small number of wells (well f, Fig. 1, step 1). Sample seeded which did not contain any nucleic acid molecules (wells a-e, Fig. 1) can then be supplemented with the dilution solution (step 3, Fig. 1) and quantitative PCR can be performed again. As illustrated in Fig, 1, step 4, readout of the fluorescence shows the presence of amplification products in an increasing number of wells (well b, c and f). The process can be repeated until substantially all the samples or wells contain an amplified population of nucleic acid molecules.

In some disclosure, specific hybridization can be used as a method for identifying and/or retrieving nucleic acids of interest. In some disclosure, solid supports, such as microparticles or beads, having a single ligand site for attachment of nucleic acid can be used to retrieve a single nucleic acid molecule. The single ligand site can be an oligonucleotide sequence complementary to part of the sequence of the nucleic acid sequences to be analyzed or complementary to an oligonucleotide tag attached to the nucleic acid sequences. Each nucleic acid of the plurality of nucleic acid sequence can be tagged with a different oligonucleotide tag or with the same oligonucleotide tag. For example, the nucleic acid sequences of the plurality of nucleic acid sequences can be ligated to a single oligonucleotide tag. Yet in other disclosure herein, a plurality oligonucleotide tags are provided. In some disclosure herein, each oligonucleotide tag has the same length. In some disclosure herein, the length of the oligonucleotide tag is at least 4 nucleotides long, at least 20 nucleotides long, at least 20 nucleotides long, at least 30 nucleotides long, or at least 40 nucleotides long.

In some disclosure herein, solid supports having complementary sequences immobilized thereon are provided. Complementary sequences may be sequences complementary to the oligonucleotide tag sequence or sequences complementary to a portion of the plurality of nucleic acid sequences. Oligonucleotide sequences may be covalently or non-covalently linked to the surface of the solid support. In some disclosure herein, the solid support may be a bead, microparticle, plate, membrane, array and the like. In preferred disclosure herein, the solid support is a microparticle or a bead. Typically the size range of the bead range from 1 µm to 1000 µm diameter. The size of the beads may be chosen to facilitate the manipulation of the beads. In some disclosure herein, the complementary sequences are attached to the solid support. Yet in other disclosure herein, the sequences complementary to the oligonucleotide tags are synthesized on the solid support as disclosed herein. In some disclosure herein, the solid support is made of controlled pore glass, nucleoside-derivatized CPG, cellulose, nylon, acrylic copolymers, dextran, polystyrene, magnetic beads and the like. In some disclosure herein, the solid support is porous. Typically, the beads have a pore size ranging from 500 to 1000 angstrom. Yet in other disclosure herein, the solid support is non porous.

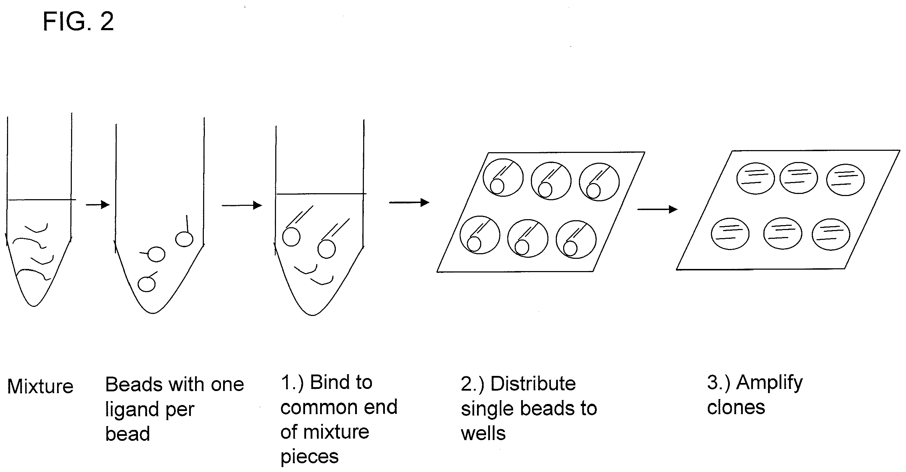

In preferred disclosure herein, a population of beads each bead having thereon a single complementary sequence to the oligonucleotide tag can be used such as each bead of the population is capable of retrieving a single nucleic acid molecule. As illustrated in Figure 2 (step 1), the nucleic acid molecules are mixed with beads comprising the single ligand site (e.g. single complementary sequence) under conditions favorable for the hybridization complementary sequences. The plurality of beads having nucleic acid sequences attached thereon may be suspended and separated into samples (e.g. in wells of a microtiter plate, Fig. 2, step 2). In some disclosure herein, the beads may be subjected to limited dilution or to the methods disclosed herein and distributed and separated such as each well contains a single bead. Nucleic acid sequences immobilized onto the isolated beads may be subjected to amplification using primers.

In some disclosure herein, the target polynucleotides are amplified after obtaining clonal populations. In some disclosure herein, the target polynucleotide may comprise universal (common to all oligonucleotides), semi-universal (common to at least a portion of the oligonucleotides) or individual or unique primer (specific to each oligonucleotide) binding sites on either the 5' end or the 3' end or both. As used herein, the term "universal" primer or primer binding site means that a sequence used to amplify the oligonucleotide is common to all oligonucleotides such that all such oligonucleotides can be amplified using a single set of universal primers. In other circumstances, an oligonucleotide contains a unique primer binding site. As used herein, the term "unique primer binding site" refers to a set of primer recognition sequences that selectively amplifies a subset of oligonucleotides. In yet other circumstances, a target nucleic acid molecule contains both universal and unique amplification sequences, which can optionally be used sequentially.

In some disclosure herein, primers/primer binding sites may be designed to be temporary. For example, temporary primers may be removed by chemical, light based or enzymatic cleavage. For example, primers/primer binding sites may be designed to include a restriction endonuclease cleavage site. In an exemplary embodiment, a primer/primer binding site contains a binding and/or cleavage site for a type IIs restriction endonuclease. In such case, amplification sequences may be designed so that once a desired set of oligonucleotides is amplified to a sufficient amount, it can then be cleaved by the use of an appropriate type IIs restriction enzyme that recognizes an internal type IIs restriction enzyme sequence of the oligonucleotide. In some disclosure herein, after amplification, the pool of nucleic acids may be contacted with one or more endonucleases to produce double-stranded breaks thereby removing the primers/primer binding sites. In certain disclosure herein, the forward and reverse primers may be removed by the same or different restriction endonucleases. Any type of restriction endonuclease may be used to remove the primers/primer binding sites from nucleic acid sequences. A wide variety of restriction endonucleases having specific binding and/or cleavage sites are commercially available, for example, from New England Biolabs (Beverly, Mass.).

In certain exemplary disclosure herein, a detectable label can be used to detect one or more nucleotides and/or oligonucleotides described herein. In some disclosure herein, detectable label is used to detect amplified molecules, for example, after quantitative PCR. Examples of detectable markers include various radioactive moieties, enzymes, prosthetic groups, fluorescent markers, luminescent markers, bioluminescent markers, metal particles, protein-protein binding pairs, protein-antibody binding pairs and the like. Examples of fluorescent proteins include, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride, phycoerythrin and the like. Examples of bioluminescent markers include, but are not limited to, luciferase (e.g., bacterial, firefly, click beetle and the like), luciferin, aequorin and the like. Examples of enzyme systems having visually detectable signals include, but are not limited to, galactosidases, glucorimidases, phosphatases, peroxidases, cholinesterases and the like. Identifiable markers also include radioactive compounds such as 1251, 35S, 14C, or 3H. Identifiable markers are commercially available from a variety of sources. Preferably the oligonucleotide probes or nucleotides are fluorescently labeled with four different fluorophores, each fluorophore being associated to a particular base or nucleotide.

In some aspects of the disclosure, the sequence of one or more nucleic acid molecules is determined and/or the nucleic acid molecules having the correct sequences of interest are selectively isolated. This can typically accomplished by conventional or next generation primary sequencing. In some disclosure herein, methods to sequence verify nucleic acid sequences using high throughput sequencing are provided. In some disclosure herein, nucleic acid molecules are sequenced by synthesis. Sequence determinations can be made by any available method permitting the querying of the sequence of an individual molecule ("single molecule sequencing"), whether directly or through the querying of an amplified population of nucleic acids derived from a single molecule ("polony sequencing"). The method of sequence determination can be non-destructive, to the extent that the objective of the sequence determination is the identification of a subsequently useful oligonucleotide. Methods of polymerase amplification and sequencing are described, for example, in U.S. Patent Application Nos. 2005-0079510 and 2006-0040297; in Mitra et al., (2003) Analytical Biochemistry 320: 55-65; Shendure et al., (2005) Science 309:1728-1732; and in Margulies et al., (2005) Nature 437:376-380. As discussed in Shendure et al. (2005) Science 309:1729, polony amplification can involve, for example, in situ polonies, in situ rolling circle amplification, bridge PCR, picotiter PCR, or emulsion PCR. Generally, an oligonucleotide to be amplified is prepared to include primer binding sites, whether as part of its sequence when initially synthesized or by subsequent ligation to adaptor molecules bearing the primer binding sites. In some disclosure herein, prior to sequencing, the oligonucleotides are immobilized at distinct locations (e.g., predetermined, addressable locations or random locations) on a solid support. In the Genome Sequencer 20 System from 454 Life Sciences, for example, beads from polony amplification are deposited into wells of a fiber-optic slide. In the method of Shendure, beads from polony amplification are poured in a 5% acrylamide gel onto a glass coverslip manipulated to form a circular gel approximately 30 microns thick, giving a disordered monolayer.

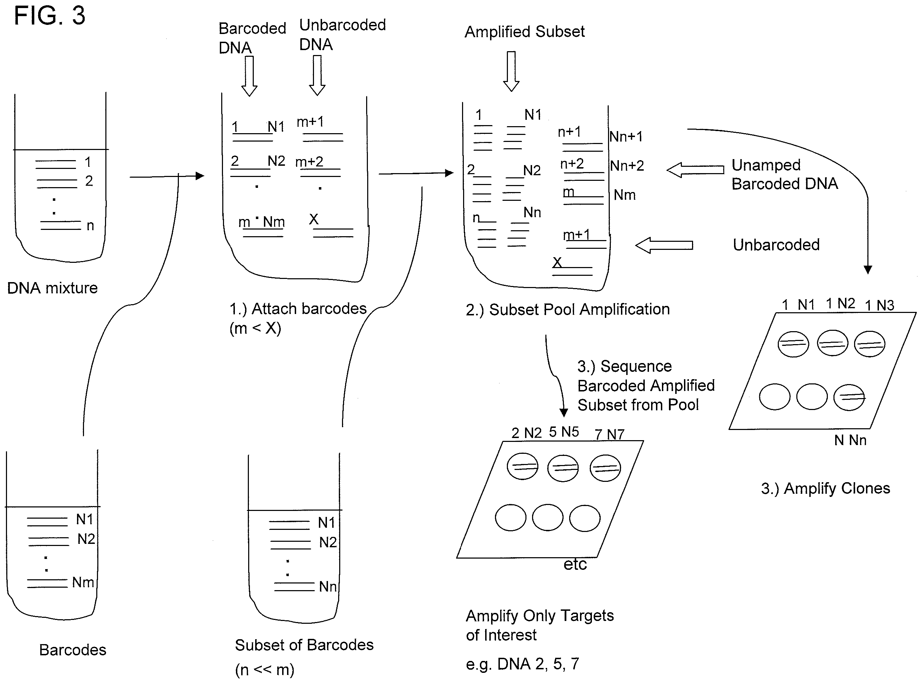

In some aspects of the disclosure, the methods involve the use of a library of identifying oligonucleotide tags (also referred herein as barcodes). In some disclosure herein, the length of the oligonucleotide tags may vary depending on the size of complexity of the nucleic acid population to be analyzed. In general, a longer oligonucleotide sequence tag will allow for a larger population of oligonucleotide tags. In some disclosure herein, each barcode differs from the other by at least 2 nucleotides. In preferred disclosure herein, a portion of the library of oligonucleotide tags is used and attached to one end terminus of the plurality of nucleic acid sequences in the population (or pool) in a stoichiometry such that a small sampling (i.e. subpopulation) of the plurality of nucleic acids in the population is bar-coded. For example, and as illustrated in Figure 3, the nucleic acid population may contain X nucleic acid molecules and a plurality m of nucleic acid tags can be provided wherein m inferior to X. After attachment of the oligonucleotide tags to the nucleic acid molecules, the resulting sub-population will comprise m bar-coded nucleic acid molecules, the remaining nucleic acid molecules being free of oligonucleotide tags ("unbar-coded") . In some disclosure herein, a number of primers n specific to a known set n of specific barcode sequences can be added, wherein n is inferior to m. Addition of primers specific to the oligonucleotide tags or barcode under favorable conditions results in the hybridization of the primers to their specific biding sites and allows for specific amplification of a subset n of the bar-coded nucleic acid sequences.

In some disclosure herein, the barcode or oligonucleotide tags are attached to the end terminus of a sub-population population of nucleic acid sequences thereby forming nucleic acid sequences-oligonucleotide tag conjugates. For example, the barcode or oligonucleotide tag can be attached using TA cloning technique. This technique relies on the ability of adenine (A) and thymine (T) on different nucleic acid sequences (e.g. oligonucleotide tag sequences and nucleic acid sequences of interest) to hybridize and ligate together in presence of ligase. In some disclosure herein, the oligonucleotide tag sequences or the nucleic acid sequences to be analyzed are amplified using a Taq polymerase which preferentially adds a A at the 3' end. In some disclosure herein, the sequences comprising a A at the 3' end are hybridized and ligated to sequences comprising a T at the 5' end.

Because of the size of the library of barcodes, each barcode sequence is expected to appear only once in the subpopulation, thus ensuring clonality. The bar-coded subpopulation may then be amplified using primers for a known set of specific barcode sequences (i.e. a subset pool), which represents a desired sampling (or subset) of the subpopulation of nucleic acids.

At this point, this amplified subset population may then be separated into samples (e.g. aliquoted into wells) and amplified. In some disclosure herein, primers specific to the individual barcode sequence can be used to preparatively clone an individual molecule from the original population of nucleic acid molecules. One skilled in the art would appreciate that the efficiency of the direct cloning methods disclosed herein may be limited by Poisson statistics due to the balance of the probability of any specific barcode appearing exactly once as a ligated product to a nucleic acid molecule from the mixture. To increase the efficiency well above Poisson statistics, other manipulations may be performed, for example sequencing the amplified subset population. Because the attached barcode can be used for multiplex sequencing on a high throughput next generation sequencing, the sequences of each nucleic acid molecule from the mixture as well as its attached barcode can be determined. The sequencing data may then be used to determine target molecules of interest from the mixture, as well as the identity of which barcodes they are attached to in the subset pool. These target molecules may then selectively amplified from the subset pool in individual wells, clonally separating them from non-target sequences in the mixture.

## Claims

1. A method of isolating a target nucleic acid having a predefined sequence, the method comprising:
(a) providing a population of X nucleic acid molecules, said population comprising a plurality of distinct nucleic acid molecules and further comprising the target nucleic acid having the predefined sequence;
(b) providing a plurality of m oligonucleotides, wherein m is inferior to X; and the oligonucleotides in the plurality are oligonucleotide tags having different sequences,
(c) attaching the oligonucleotide tags to one terminus of nucleic acid molecules of the population, thereby generating a subpopulation of tagged molecules, each tagged molecule in the sub-population having a different oligonucleotide tag, wherein an oligonucleotide tag is attached to the target nucleic acid having the predefined sequence;
(d) amplifying the plurality of tagged molecules using primers complementary to the plurality of oligonucleotide tags;
(e) sequencing the amplified plurality of tagged molecules;
(f) identifying at least one target tagged molecule having the predefined sequence; and
(g) isolating the at least one target tagged molecule having the predefined sequence.

2. The method of claim 1 further comprising sequencing the at least one isolated target tagged molecule.

3. The method of claim 1 or claim 2 further comprising removing the oligonucleotide tag to recover the target nucleic acid.

4. The method of claim 2 wherein the sequencing step is by high throughput sequencing.

5. The method of claim 2 further comprising amplifying the at least one target tagged molecule.

6. A method of isolating a target nucleic acid having a predefined sequence, the method comprising:
(a) providing a population of X nucleic acid molecules, said population comprising a plurality of different nucleic acid molecules and further comprising the target nucleic acid having the predefined sequence,
(b) attaching a plurality of m oligonucleotides to nucleic acid molecules of the population, wherein the oligonucleotides in the plurality are oligonucleotide tags having different sequences and m is inferior to X, to generate a sub-population of tagged nucleic acid molecules, each nucleic acid molecule in the sub-population having a different oligonucleotide tag attached thereto; wherein an oligonucleotide tag is attached to the target nucleic acid having the predefined sequence;
(c) providing a plurality of microparticles, wherein each microparticle has, immobilized on its surface, an oligonucleotide sequence complementary to a portion of the nucleic acid molecules; and the complementary oligonucleotide sequences immobilized to microparticles of the plurality are different,
(d) mixing the sub-population from step (b) and the plurality of microparticles from step (c), and hybridizing the nucleic acid molecules to the complementary oligonucleotide sequences on the microparticles;
(e) sequencing the nucleic acid molecules;
(f) identifying at least one target nucleic acid molecule having the predefined sequence; and
(g) isolating the at least one target nucleic acid molecule having the predefined sequence.

7. The method of claim 6 wherein each microparticle has a single complementary oligonucleotide sequence on its surface.

8. The method of claim 7 wherein the each tagged nucleic acid molecule in the sub-population of step (b) comprises a different oligonucleotide tag at one terminus.

9. The method of claim 1 or claim 6 wherein the step of isolating is by limiting dilution.

10. The method of claim 6 further comprising amplifying the at least one target molecule.

11. A method of isolating a target nucleic acid having a predefined sequence, the method comprising:
(a) providing a population of X nucleic acid molecules, said population comprising a plurality of different nucleic acid molecules and further comprising the target nucleic acid having the predefined sequence,
(b) attaching a plurality of m oligonucleotides to nucleic acid molecules of the population, wherein the oligonucleotides in the plurality are oligonucleotide tags having different sequences and m is inferior to X, to generate a sub-population of tagged nucleic acid molecules, each nucleic acid molecule in the sub-population having a different oligonucleotide tag attached thereto; wherein an oligonucleotide tag is attached to the target nucleic acid having the predefined sequence;
(c) providing a dilution of the sub-population of nucleic acid molecules;
(d) separating the nucleic acid molecules into samples each comprising a single nucleic acid molecule or no nucleic acid molecules;
(e) amplifying the separated nucleic acid molecules from step (d) thereby forming amplified nucleic acid molecules;
(f) repeating steps (c), (d) and (e) in nucleic acid free samples;
(g) sequencing the amplified nucleic acid molecules;
(h) identifying at least one amplified nucleic acid molecule having a predefined sequence; and
(i) isolating the least one target nucleic acid molecule having the predefined sequence.

12. The method of claim 6 or 12 further comprising sequencing the at least one target molecule.

## Patentansprüche

1. Verfahren zum Isolieren einer Ziel-Nucleinsäure mit einer vordefinierten Sequenz, wobei das Verfahren Folgendes umfasst:
(a) Bereitstellen einer Population von X Nucleinsäuremolekülen, wobei die Population eine Vielzahl von unterschiedlichen Nucleinsäuremolekülen umfasst und weiters die Ziel-Nucleinsäure mit der vordefinierten Sequenz umfasst;
(b) Bereitstellen einer Vielzahl von m Oligonucleotiden, wobei m kleiner als X ist; und die Oligonucleotide in der Vielzahl Oligonucleotidmarkierungen mit unterschiedlichen Sequenzen sind,
(c) Anbringen der Oligonucleotidmarkierungen an einen Terminus der Nucleinsäuremoleküle der Population, wodurch eine Unterpopulation von markierten Molekülen erzeugt wird, wobei jedes markierte Molekül in der Unterpopulation eine unterschiedliche Oligonucleotidmarkierung aufweist, wobei eine Oligonucleotidmarkierung an die Ziel-Nucleinsäure mit der vordefinierten Sequenz angebracht wird;
(d) Amplifizieren der Vielzahl von markierten Molekülen unter Verwendung von Primern, die zur Vielzahl von Oligonucleotidmarkierungen komplementär sind;
(e) Sequenzieren der amplifizierten Vielzahl markierter Moleküle;
(f) Identifizieren von zumindest einem markierten Ziel-Molekül mit der vordefinierten Sequenz; und
(g) Isolieren des zumindest einen markierten Ziel-Moleküls mit der vordefinierten Sequenz.

2. Verfahren nach Anspruch 1, das weiters das Sequenzieren des zumindest einen isolierten markierten Ziel-Moleküls umfasst.

3. Verfahren nach Anspruch 1 oder Anspruch 2, das weiters das Entfernen der Oligonucleotidmarkierung umfasst, um die Ziel-Nucleinsäure zu gewinnen.

4. Verfahren nach Anspruch 2, wobei der Sequenzierschritt mittels Hochdurchsatz-Sequenzieren erfolgt.

5. Verfahren nach Anspruch 2, das weiters das Amplifizieren des zumindest einen markierten Ziel-Moleküls umfasst.

6. Verfahren zum Isolieren einer Ziel-Nucleinsäure mit einer vordefinierten Sequenz, wobei das Verfahren Folgendes umfasst:
(a) Bereitstellen einer Population von X Nucleinsäuremolekülen, wobei die Population eine Vielzahl von unterschiedlichen Nucleinsäuremolekülen umfasst und weiters die Ziel-Nucleinsäure mit der vordefinierten Sequenz umfasst;
(b) Anbringen einer Vielzahl von m Oligonucleotiden an Nucleinsäuremoleküle der Population, wobei die Oligonucleotide in der Vielzahl Oligonucleotidmarkierungen mit unterschiedlichen Sequenzen sind und m kleiner als X ist, um eine Unterpopulation von markierten Nucleinsäuremolekülen zu erzeugen, wobei jedes Nucleinsäuremolekül in der Unterpopulation eine unterschiedliche Oligonucleotidmarkierung aufweist, die daran angebracht ist; wobei eine Oligonucleotidmarkierung an der Ziel-Nucleinsäure mit der vordefinierten Sequenz angebracht wird;
(c) Bereitstellen einer Vielzahl von Mikropartikeln, wobei jedes Mikropartikel eine auf seiner Oberfläche immobilisierte Oligonucleotidsequenz aufweist, die zu einem Teil der Nucleinsäuremoleküle komplementär ist; und die komplementären Oligonucleotidsequenzen, die an Mikropartikel der Vielzahl immobilisiert sind, unterschiedlich sind,
(d) Mischen der Unterpopulation aus Schritt (b) und der Vielzahl von Mikropartikeln aus Schritt (c) und Hybridisieren der Nucleinsäuremoleküle an die komplementären Oligonucleotidsequenzen auf den Mikropartikeln;
(e) Sequenzieren der Nucleinsäuremoleküle;
(f) Identifizieren von zumindest einem Ziel-Nucleinsäuremolekül mit der vordefinierten Sequenz; und
(g) Isolieren des zumindest einen Ziel-Nucleinsäuremoleküls mit der vordefinierten Sequenz.

7. Verfahren nach Anspruch 6, wobei jedes Mikropartikel eine einzelne komplementäre Oligonucleotidsequenz auf seiner Oberfläche aufweist.

8. Verfahren nach Anspruch 7, wobei jedes markierte Nucleinsäuremolekül in der Unterpopulation aus Schritt (b) eine unterschiedliche Oligonucleotidmarkierung an einem Terminus umfasst.

9. Verfahren nach Anspruch 1 oder Anspruch 6, wobei der Schritt des Isolierens mittels Grenzverdünnung erfolgt.

10. Verfahren nach Anspruch 6, das weiters das Amplifizieren des zumindest einen Ziel-Moleküls umfasst.

11. Verfahren zum Isolieren einer Ziel-Nucleinsäure mit einer vordefinierten Sequenz, wobei das Verfahren Folgendes umfasst:
(a) Bereitstellen einer Population von X Nucleinsäuremolekülen, wobei die Population eine Vielzahl von unterschiedlichen Nucleinsäuremolekülen umfasst und weiters die Ziel-Nucleinsäure mit der vordefinierten Sequenz umfasst;
(b) Anbringen einer Vielzahl von m Oligonucleotiden an Nucleinsäuremoleküle der Population, wobei die Oligonucleotide in der Vielzahl Oligonucleotidmarkierungen mit unterschiedlichen Sequenzen sind und m kleiner als X ist, um eine Unterpopulation von markierten Nucleinsäuremolekülen zu erzeugen, wobei jedes Nucleinsäuremolekül in der Unterpopulation eine unterschiedliche Oligonucleotidmarkierung aufweist, die daran angebracht ist; wobei eine Oligonucleotidmarkierung an die Ziel-Nucleinsäure mit der vordefinierten Sequenz angebracht ist;
(c) Bereitstellen einer Verdünnung der Unterpopulation von Nucleinsäuremolekülen;
(d) Trennen der Nucleinsäuremoleküle in Proben, die jeweils ein einzelnes Nucleinsäuremolekül oder kein Nucleinsäuremolekül umfassen;
(e) Amplifizieren der getrennten Nucleinsäuremoleküle aus Schritt (d), wodurch amplifizierte Nucleinsäuremoleküle gebildet werden;
(f) Wiederholen der Schritte (c), (d) und (e) in nucleinsäurefreien Proben;
(g) Sequenzieren der amplifizierten Nucleinsäuremoleküle;
(h) Identifizieren von zumindest einem amplifizierten Nucleinsäuremolekül mit der vordefinierten Sequenz; und
(i) Isolieren des zumindest einen Ziel-Nucleinsäuremoleküls mit der vordefinierten Sequenz.

12. Verfahren nach Anspruch 6 oder 12, das weiters das Sequenzieren des zumindest einen Ziel-Moleküls umfasst.

## Revendications

1. Procédé d'isolement d'un acide nucléique cible possédant une séquence prédéfinie, le procédé comprenant :
(a) la mise à disposition d'une population de X molécules d'acide nucléique, ladite population comprenant une pluralité de molécules d'acide nucléique distinctes et comprenant en outre l'acide nucléique cible possédant la séquence prédéfinie ;
(b) la mise à disposition d'une pluralité de m oligonucléotides, où m est inférieur à X ; et les oligonucléotides dans la pluralité sont des marqueurs oligonucléotidiques possédant des séquences différentes,
(c) l'attachement des marqueurs oligonucléotidiques à une seule extrémité des molécules d'acide nucléique de la population, en générant ainsi une sous-population de molécules marquées, chaque molécule marquée dans la sous-population possédant un marqueur oligonucléotidique différent, où un marqueur oligonucléotidique est attaché à l'acide nucléique cible possédant la séquence prédéfinie ;
(d) l'amplification de la pluralité de molécules marquées en utilisant des amorces complémentaires à la pluralité de marqueurs oligonucléotidiques ;
(e) le séquençage de la pluralité de molécules marquées amplifiée ;
(f) l'identification d'au moins une molécule marquée cible possédant la séquence prédéfinie ; et
(g) l'isolement de la au moins une molécule marquée cible possédant la séquence prédéfinie.

2. Procédé selon la revendication 1, comprenant en outre le séquençage de la au moins une molécule marquée cible isolée.

3. Procédé selon la revendication 1 ou la revendication 2, comprenant en outre l'élimination du marqueur oligonucléotidique afin de récupérer l'acide nucléique cible.

4. Procédé selon la revendication 2, dans lequel l'étape de séquençage est réalisée par un séquençage à haut débit.

5. Procédé selon la revendication 2, comprenant en outre l'amplification de la au moins une molécule marquée cible.

6. Procédé d'isolement d'un acide nucléique cible possédant une séquence prédéfinie, le procédé comprenant :
(a) la mise à disposition d'une population de X molécules d'acide nucléique, ladite population comprenant une pluralité de molécules d'acide nucléique différentes et comprenant en outre l'acide nucléique cible possédant la séquence prédéfinie,
(b) l'attachement d'une pluralité de m oligonucléotides aux molécules d'acide nucléique de la population, où les oligonucléotides dans la pluralité sont des marqueurs oligonucléotidiques possédant des séquences différentes et m est inférieur à X, afin de générer une sous-population de molécules d'acide nucléique marquées, chaque molécule d'acide nucléique marquée dans la sous-population possédant un marqueur oligonucléotidique différent attaché à celle-ci ; où un marqueur oligonucléotidique est attaché à l'acide nucléique cible possédant la séquence prédéfinie ;
(c) la mise à disposition d'une pluralité de microparticules, dans laquelle chaque microparticule possède, immobilisée sur sa surface, une séquence oligonucléotidique complémentaire à une partie des molécules d'acide nucléique ; et les séquences oligonucléotidiques complémentaires immobilisées sur les microparticules de la pluralité sont différentes,
(d) le mélange de la sous-population de l'étape (b) et de la pluralité de microparticules de l'étape (c), et l'hybridation des molécules d'acide nucléique aux séquences oligonucléotidiques complémentaires sur les microparticules ;
(e) le séquençage des molécules d'acide nucléique ;
(f) l'identification d'au moins une molécule d'acide nucléique cible possédant la séquence prédéfinie ; et
(g) l'isolement de la au moins une molécule d'acide nucléique cible possédant la séquence prédéfinie.

7. Procédé selon la revendication 6, dans lequel chaque microparticule possède une seule séquence oligonucléotidique complémentaire sur sa surface.

8. Procédé selon la revendication 7, dans lequel la chaque molécule d'acide nucléique marquée dans la sous-population de l'étape (b) comprend un marqueur oligonucléotidique différent à une seule extrémité.

9. Procédé selon la revendication 1 ou la revendication 6, dans lequel l'étape d'isolement est réalisée par dilution limite.

10. Procédé selon la revendication 6, comprenant en outre l'amplification de la au moins une molécule cible.

11. Procédé d'isolement d'un acide nucléique cible possédant une séquence prédéfinie, le procédé comprenant :
(a) la mise à disposition d'une population de X molécules d'acide nucléique, ladite population comprenant une pluralité de molécules d'acide nucléique différentes et comprenant en outre l'acide nucléique cible possédant la séquence prédéfinie,
(b) l'attachement d'une pluralité de m oligonucléotides aux molécules d'acide nucléique de la population, où les oligonucléotides dans la pluralité sont des marqueurs oligonucléotidiques possédant des séquences différentes et m est inférieur à X, afin de générer une sous-population de molécules d'acide nucléique marquées, chaque molécule d'acide nucléique dans la sous-population possédant un marqueur oligonucléotidique différent attaché à celle-ci ; où un marqueur oligonucléotidique est attaché à l'acide nucléique cible possédant la séquence prédéfinie ;
(c) la mise à disposition d'une dilution de la sous-population de molécules d'acide nucléique ;
(d) la séparation des molécules d'acide nucléique en échantillons comprenant chacun une seule molécule d'acide nucléique ou aucune molécule d'acide nucléique ;
(e) l'amplification des molécules d'acide nucléique séparées de l'étape (d) en formant ainsi des molécules d'acide nucléique amplifiées ;
(f) la répétition des étapes (c), (d) et (e) dans des échantillons exempts d'acide nucléique ;
(g) le séquençage des molécules d'acide nucléique amplifiées ;
(h) l'identification d'au moins une molécule d'acide nucléique amplifiée possédant une séquence prédéfinie ; et
(i) l'isolement de la au moins une molécule d'acide nucléique cible possédant la séquence prédéfinie.

12. Procédé selon la revendication 6 ou 12, comprenant en outre le séquençage de la au moins une molécule cible.
